(19) Europäisches Patentamt / European Patent Office / Office européen des brevets

(11) **EP 4 098 980 A1**

(12) **EUROPEAN PATENT APPLICATION**
published in accordance with Art. 153(4) EPC

(43) Date of publication:
07.12.2022 Bulletin 2022/49

(21) Application number: 21746930.3

(22) Date of filing: 27.01.2021

(51) International Patent Classification (IPC):
$G01F\ 1/66^{(2022.01)}$  $A61B\ 5/026^{(2006.01)}$
$A61B\ 5/0285^{(2006.01)}$  $G01F\ 1/00^{(2022.01)}$

(52) Cooperative Patent Classification (CPC):
A61B 5/026; A61B 5/0285; G01F 1/00; G01F 1/66

(86) International application number:
PCT/JP2021/002743

(87) International publication number:
WO 2021/153582 (05.08.2021 Gazette 2021/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.01.2020 JP 2020013594

(71) Applicant: Kyocera Corporation
Kyoto-shi Kyoto 612-8501 (JP)

(72) Inventors:
• MATSUNAGA, Shougo
Kyoto-shi, Kyoto 612-8501 (JP)
• TODA, Keisuke
Kyoto-shi, Kyoto 612-8501 (JP)

(74) Representative: Viering, Jentschura & Partner
mbB
Patent- und Rechtsanwälte
Am Brauhaus 8
01099 Dresden (DE)

(54) **MEASUREMENT DEVICE**

(57) A first shield case covers an amplifier on a second surface of a wiring board. A sensor on a first surface of the wiring board includes a light emitter that illuminates, with light, an illumination target having an internal space in which a fluid flows, a light receiver that receives coherent light including light scattered by the illumination target in the light from the light emitter and outputs an output signal corresponding to intensity of the coherent light, and a package accommodating the light emitter and the light receiver and including a first wire. The amplifier amplifies the output signal. The calculator calculates a calculation value indicating a flow state of the fluid based on the output signal amplified by the amplifier. The first wire surrounds the light emitter and the light receiver in a plan view or in a transparent plan view of the package. The first wire and the first shield case are electrically connected to a ground wire.

FIG. 2

EP 4 098 980 A1

## Description

### FIELD

The present disclosure relates to a measurement device.

### BACKGROUND

Patent Literature 1 describes a technique for improving the noise immunity of integrated circuits. Patent Literature 2 describes a technique for improving the noise immunity of communication modules.

CITATION LIST

PATENT LITERATURE

Patent Literature 1: Japanese Unexamined Patent Application Publication No. 6-350280
Patent Literature 2: Japanese Unexamined Patent Application Publication No. 10-233471

### BRIEF SUMMARY

A measurement device according to one embodiment of the present disclosure includes a wiring board having a first surface and a second surface opposite to the first surface and including a ground wire, a sensor on the first surface, a calculator, an amplifier on the second surface, and a first shield case covering the amplifier. The sensor includes a light emitter that illuminates, with light, an illumination target having an internal space in which a fluid flows, a light receiver that receives coherent light including light scattered by the illumination target in the light from the light emitter, and outputs an output signal corresponding to intensity of the coherent light, and a package accommodating the light emitter and the light receiver and including a first wire. The amplifier amplifies the output signal. The calculator calculates a calculation value indicating a flow state of the fluid based on the output signal amplified by the amplifier. The first wire surrounds the light emitter and the light receiver in a plan view or in a transparent plan view of the package. The first wire and the first shield case are electrically connected to the ground wire.

### BRIEF DESCRIPTION OF DRAWINGS

FIG. 1 is a perspective view of a measurement device showing an example appearance.
FIG. 2 is a cross-sectional view of the measurement device.
FIG. 3 is a cross-sectional view of the measurement device.
FIG. 4 is a plan view of an example shield case and an example wiring board.
FIG. 5 is a graph showing an example power spectrum.
FIG. 6 is a plan view of an example sensor.
FIG. 7 is a plan view of an example inner-layer wire.
FIG. 8 is a plan view of an example inner-layer wire.
FIG. 9 is a plan view of an example wire including multiple layers in a package.
FIG. 10 is a plan view of an example wire including multiple layers in a package.
FIG. 11 is a plan view of an example wire including multiple layers in a package.
FIG. 12 is a plan view of an example shield case and an example wiring board.
FIG. 13 is a graph showing an example power spectrum.
FIG. 14 is a cross-sectional view of an example measurement device.
FIG. 15 is a cross-sectional view of an example measurement device.

### DETAILED DESCRIPTION

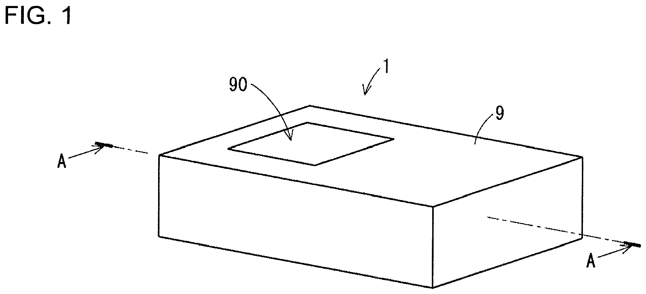

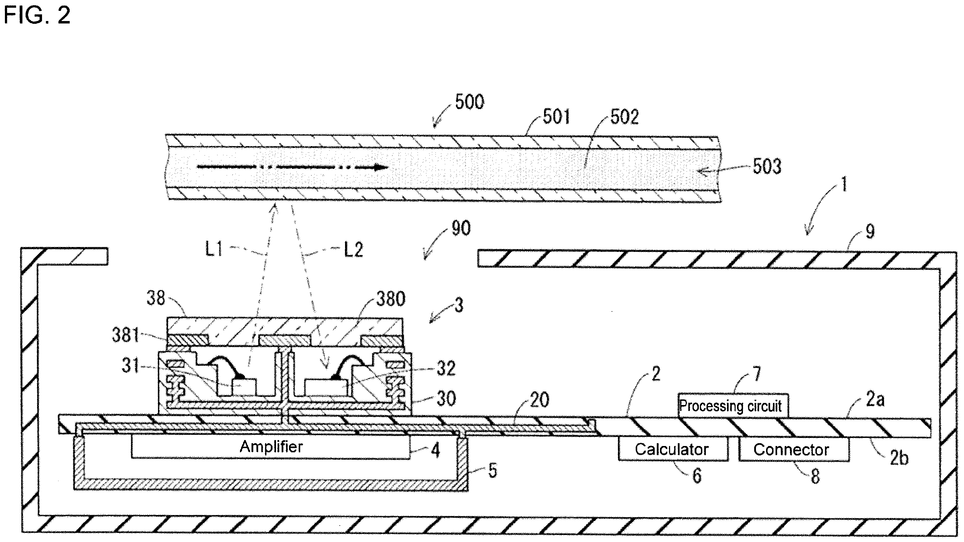

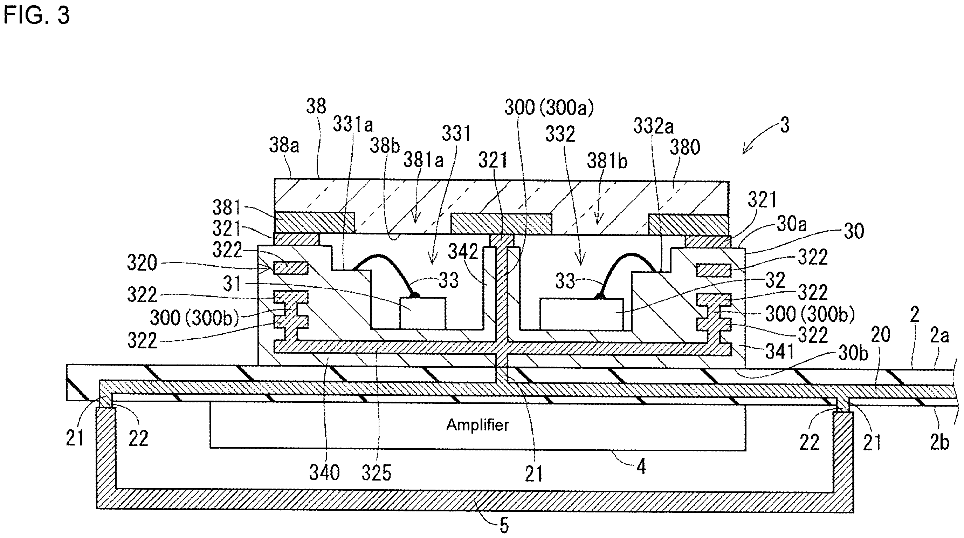

FIG. 1 is a schematic perspective view of a measurement device 1 showing an example appearance. FIG. 2 is a schematic cross-sectional view of the measurement device 1 taken along line A-A in FIG. 1 as viewed in the direction indicated by arrows. FIG. 3 is a partially enlarged cross-sectional view of the measurement device 1 shown in FIG. 2.

The components of the measurement device 1 will be hereafter described with upper parts of FIGs. 2 and 3 corresponding to an upper part of the measurement device 1 and lower parts of FIGs. 2 and 3 corresponding to a lower part of the measurement device 1.

The measurement device 1 can illuminate, with light L1, an object (also referred to as an illumination target) 500 having an internal space 503 in which a fluid 502 flows, as shown in FIG. 2. The illumination target 500 includes an object (flow passage component) 501 defining a flow passage, and the fluid 502 flowing through the flow passage. The internal space 503 of the flow passage component 501 is the internal space 503 of the illumination target 500. The measurement device 1 can receive coherent light including light scattered by the illumination target 500 and quantitatively determine the flow state of the fluid 502 based on the received coherent light. In other words, the measurement device 1 can determine a quantitative value (also referred to as a flow quantitative value) indicating the flow state of the fluid 502. The flow passage component 501 includes, for example, a tubular object (also referred to as a tubular body) such as a blood vessel in a living body or pipes in various devices. The flow quantitative value includes, for example, at least one of a flow rate or a flow velocity. The flow rate is, for example, the quantity of a fluid that passes through the flow passage per unit time. The quantity of the fluid is expressed in, for example, a volume or mass. The flow velocity is the velocity of the fluid that passes through the flow passage. The flow velocity is expressed with, for example, a distance by which the fluid flows per unit time.

The measurement device 1 can quantitatively determine the flow state of the fluid 502 with, for example, the Doppler effect for light. When, for example, light illuminating the fluid 502 is scattered by the fluid 502, the Doppler effect corresponding to the flow of the fluid 502 causes a shift (also referred to as a Doppler shift) of the frequency of light corresponding to the movement speed of the fluid 502. The measurement device 1 can quantitatively determine the flow state of the fluid 502 with this Doppler shift.

The fluid 502 is a target (also referred to as a measurement target) for which the flow state is determined quantitatively. For example, the fluid 502 may scatter light. In some embodiments, the fluid 502 may be an object that carries a substance (also referred to as a scatter substance) that scatters light or an object (also referred to as a scatterer) that scatters light. Examples of the fluid 502 serving as a measurement target include water, blood, printer ink, or gas containing a scatterer such as powder. When a scatter substance or a scatterer flows with the fluid, the flow rate of the scatter substance or the scatterer may be used as the flow rate of the fluid, or the flow velocity of the scatter substance or the scatterer may be used as the flow velocity of the fluid.

As shown in FIGs. 1 to 3, the measurement device 1 includes, for example, a wiring board 2, a sensor 3, an amplifier 4, a shield case 5, a calculator 6, a processing circuit 7, a connector 8, and an outer case 9.

The outer case 9 accommodates the wiring board 2, the sensor 3, the amplifier 4, the shield case 5, the calculator 6, the processing circuit 7, and the connector 8. The outer case 9 is, for example, a box. The outer case 9 is, for example, substantially rectangular or cubic. The outer case 9 is formed from, for example, a resin.

The wiring board 2 includes a surface 2a and a surface 2b opposite to the surface 2a. When, for example, the wiring board 2 is a flat plate, the surface 2a may be an upper surface and the surface 2b may be a lower surface. The wiring board 2 includes multiple wires including a ground wire 20. The wiring board 2 is, for example, a multilayer board. The ground wire 20 is formed, for example, in an inner layer of the wiring board 2. The ground wire 20 may be a wire extending in a planar manner along the surfaces 2a and 2b, or a wire in another pattern. The wiring board 2 includes multiple vias that electrically connect multiple layers of a wire to each other, and multiple connection pads located on the surfaces 2a and 2b. The vias include multiple vias 21 connected to the ground wire 20 (refer to FIG. 3). The connection pads include multiple connection pads 22 electrically connected to the ground wire 20 (refer to FIG. 3). Each of the vias 21 electrically connects the corresponding connection pad 22 to the ground wire 20.

The sensor 3 and the processing circuit 7 are located, for example, on the surface 2a. The amplifier 4, the shield case 5, the calculator 6, and the connector 8 are located, for example, on the surface 2b.

The wiring board 2 may be a single-layer board. The processing circuit 7 may be located on the surface 2b. The amplifier 4 and the shield case 5 may be located on the surface 2a. At least one of the calculator 6 or the connector 8 may be located on the surface 2a.

For example, the connector 8 is connected to a cable extending from a device external to the measurement device 1. This allows the measurement device 1 to be electrically connected to the external device. For example, power from a power supply for the measurement device 1 is supplied to the connector 8. The power supplied to the connector 8 is then supplied to the amplifier 4, the calculator 6, the processing circuit 7, and other circuits through a power supply wire in the wiring board 2. The connector 8 may electrically connect the ground wire 20 in the wiring board 2 to a metal housing or a chassis included in the device external to the measurement device 1. The ground wire 20 may be grounded through the connector 8.

The sensor 3 includes, for example, a light emitter 31 and a light receiver 32. As shown in FIG. 2, the light emitter 31 can illuminate, with the light L1, the illumination target 500. The outer case 9 has an opening 90 facing the sensor 3. Through the opening 90, the light L1 is applied to the illumination target 500. Examples of the light L1 emitted from the light emitter 31 to illuminate the illumination target 500 include light having a predetermined wavelength suitable for the fluid 502. For blood serving as an example of the fluid 502, the light L1 may have a wavelength set to about 600 to 900 nm. For printer ink serving as an example of the fluid 502, the light L1 may have a wavelength set to about 700 to 1000 nm. A semiconductor laser device such as a vertical-cavity surface-emitting laser (VCSEL) is usable as an example of the light emitter 31.

The light receiver 32 can receive coherent light L2 including light scattered by the illumination target 500 in the light L1 emitted from the light emitter 31 to illuminate the illumination target 500. The light receiver 32 receives the coherent light L2 through the opening 90 of the outer case 9. For example, the light receiver 32 can convert the received light to an electric signal corresponding to the light intensity. In other words, the light receiver 32 can receive the coherent light L2 including light scattered by the illumination target 500, and output an output signal corresponding to the intensity of the coherent light L2. The coherent light L2 that can be received by the light receiver 32 includes coherent light, in the light scattered by the illumination target 500, caused by scattered light without a Doppler shift from an object stationary around the fluid 502 (also referred to as a stationary object) and scattered light with a Doppler shift from the fluid 502. For blood flowing through a blood vessel serving as an example of the fluid 502, the stationary object includes the skin and the blood vessels. For ink flowing through a pipe serving as an example of the fluid 502, the stationary object includes the pipe. In this case, the pipe is formed from, for example, a light-transmissive material. Examples of the light-transmissive material include a glass and a polymer resin.

A change in the intensity of the coherent light L2 with time (also referred to as a temporal change) indicates a beat of the frequency corresponding to a frequency difference between the frequency of scattered light without a Doppler shift and the frequency of scattered light with a Doppler shift. Thus, the output signal from the light receiver 32 corresponding to the intensity of the coherent light L2 includes a component of a signal corresponding to the beat (also referred to as a beat signal or an optical beat signal) with respect to the temporal change in the intensity of the coherent light L2. An example of the light receiver 32 is a device that can follow the beat (also referred to as having time resolution) with respect to the temporal change in the intensity of the coherent light L2. The wavelength of light receivable by the light receiver 32 can be set in accordance with the measurement conditions such as the wavelength range of the light L1 emitted from the light emitter 31 to illuminate the illumination target 500 and the velocity range of the fluid 502. Examples of the light receiver 32 include various photodiodes including a silicon (Si) photodiode, a gallium arsenide (GaAs) photodiode, an indium gallium arsenide (InGaAs) photodiode, and a germanium (Ge) photodiode.

The sensor 3 includes, for example, a package 30 and a cover 38, in addition to the light emitter 31 and the light receiver 32. The package 30 accommodates the light emitter 31 and the light receiver 32. The package 30 has a recess that accommodates the light emitter 31 and a recess that accommodates the light receiver 32. The cover 38 covers the recesses in the package 30. The components of the package 30 and the cover 38 will be described in detail later.

The wiring board 2 electrically connects the amplifier 4 to the light receiver 32. The amplifier 4 amplifies and outputs the output signal from the light receiver 32. The amplifier 4 includes, for example, an operational amplifier, a resistor, and a capacitor. The vias 21 and the connection pads 22 on the surface 2b electrically connect a ground terminal in the amplifier 4 to the ground wire 20 in the wiring board 2. Hereafter, the output signal simply refers to the output signal from the light receiver 32.

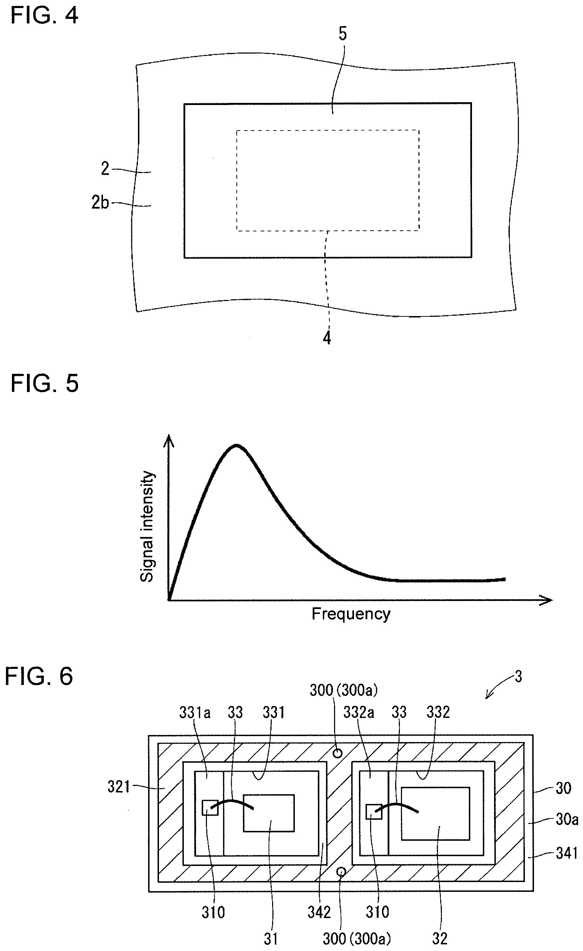

FIG. 4 is a schematic view of the shield case 5 and the wiring board 2 in a plan view from below. The shield case 5 is, for example, a box, as shown in FIGs. 2 to 4. The shield case 5 is formed from, for example, a metal such as aluminum or stainless steel. The shield case 5 is located on the surface 2b of the wiring board 2 to cover the amplifier 4. The vias 21 and the connection pads 22 on the surface 2b electrically connect the shield case 5 to the ground wire 20 in the wiring board 2. For example, the shield case 5 is soldered on the connection pads 22 on the surface 2b, and fixed to the surface 2b.

The structure of the shield case 5 is not limited to the above structure. The shield case 5 may include a base formed from, for example, a resin with a surface covered with a metal film. This metal film is formed from, for example, nickel.

For example, the processing circuit 7 includes multiple electronic components including a capacitor, a resistor, and an inductor. The wiring board 2 electrically connects the processing circuit 7 to the light receiver 32 in the sensor 3 and the amplifier 4. The vias 21 and the connection pads 22 on the surface 2a electrically connect a ground terminal in the processing circuit 7 to the ground wire 20.

The processing circuit 7 can perform, for example, multiple types of processes. For example, the processing circuit 7 can perform a filter process on the output signal amplified by the amplifier 4. After the filter process performed by the processing circuit 7, the output signal is input into the calculator 6. The processing circuit 7 can control supply of electric power to the light emitter 31 to control emission of the light emitter 31.

The wiring board 2 electrically connects the calculator 6 to the processing circuit 7. The vias 21 and the connection pads 22 on the surface 2a electrically connect a ground terminal in the calculator 6 to the ground wire 20. The calculator 6 can perform various processes on the output signal input from the processing circuit 7 after the filter process. The calculator 6 includes, for example, at least one processor. In various embodiments, the processor(s) may be a single integrated circuit (IC), multiple ICs connected to one another for mutual communication, or discrete circuits. The processor(s) may use various known technologies.

In one embodiment, the processor includes one or more circuits or units that perform one or more data computation procedures or processes by, for example, executing instructions stored in an associated memory. In other embodiments, the processor may be firmware (e.g., a discrete logic component) that performs one or more data computation procedures or processes.

In various embodiments, the processor includes one or more processors, controllers, microprocessors, microcontrollers, application-specific integrated circuits (ASICs), digital signal processors, programmable logic devices, field programmable gate arrays, combinations of any of these devices or configurations, or combinations of other known devices and configurations, and may perform the functions described below.

In the embodiment, the calculator 6 includes, for example, a microprocessor. The microprocessor includes, for example, a central processing unit (CPU), a memory circuit, and an analog-digital (A/D) converter. The memory circuit includes, for example, a non-transitory recording medium readable by the CPU such as a read-only memory (ROM) and a random-access memory (RAM). The memory circuit stores a control program and other programs. The CPU executes the control program in the memory circuit to achieve various functions of the microprocessor.

The A/D converter in the calculator 6 can convert an analog output signal input from the processing circuit 7 to a digital signal. The CPU in the calculator 6 can quantitatively determine the flow state of the fluid 502 based on the digital output signal. A method used by the calculator 6 for quantitatively determining the flow state of the fluid 502 based on the output signal from the light receiver 32 will be described below.

The frequency and the intensity (also referred to as signal intensity) of the output signal from the light receiver 32 depend on the Doppler effect for light. Thus, a power spectrum indicating the relationship between the frequency and the intensity of the output signal changes in accordance with the flow quantitative value (flow rate or flow velocity) of the fluid 502. The calculator 6 (specifically, the CPU) performs a Fourier transform (specifically, a discrete Fourier transform) of the output signal from the processing circuit 7 to determine the power spectrum of the output signal.

FIG. 5 is a graph showing an example power spectrum determined by the calculator 6. In FIG. 5, the horizontal axis represents the frequency, and the vertical axis represents the signal intensity of the output signal for each frequency.

The calculator 6 calculates a calculation value (also referred to as a flow calculation value) indicating the flow state of the fluid 502 flowing through the internal space 503 of the illumination target 500 based on the determined power spectrum.

In this calculation, fn represents frequency, and P(fn) represents the signal intensity of the output signal for the frequency fn. For example, the calculator 6 integrates the signal intensity P(fn) that is weighted using the frequency fn to determine the flow calculation value. The flow calculation value Vc is represented by Formula 1 below.

$$Vc = \sum fn \times P(fn) \qquad (1)$$

The calculator 6 calculates a quantitative value (flow quantitative value) indicating the flow state of the fluid 502 based on the determined flow calculation value Vc. For example, the calculator 6 calculates the flow quantitative value based on the calculated flow calculation value Vc and prepared calibration data (also referred to as a calibration curve). For example, the calibration data is prestored in the memory circuit in the calculator 6 before the flow quantitative value of the fluid 502 is calculated. The calibration data may be stored in the form of, for example, a functional formula or a table. When the calibration data used for the flow rate of the fluid 502 is stored in the memory circuit in the calculator 6, the calculator 6 can calculate the flow rate of the fluid 502 based on the determined flow calculation value Vc and the stored calibration data. When the calibration data used for the flow velocity of the fluid 502 is stored in the memory circuit in the calculator 6, the calculator 6 can calculate the flow velocity of the fluid 502 based on the determined flow calculation value Vc and the stored calibration data.

The calculator 6 may eliminate calculation of the flow quantitative value based on the flow calculation value. In this case, the device external to the measurement device 1 may calculate the flow quantitative value based on the flow calculation value calculated by the calculator 6. The flow calculation value calculated by the calculator 6 may be provided to the external device through the connector 8. The calculation of the flow calculation value by the calculator 6 is not limited to the above calculation.

Examples of Package and Cover in Sensor

FIG. 6 is a schematic plan view of an example structure of the sensor 3 without the cover 38. FIG. 6 schematically shows an example of the sensor 3 without the cover 38 as viewed from above the sensor 3.

Example Structure of Package

The package 30 is substantially rectangular and has six surfaces, as shown in FIGs. 2, 3, and 6. The package 30 is mounted on the surface 2a of the wiring board 2 with a surface 30b included in the six surfaces facing the surface 2a. The surface 30b is the lower surface 30b of the package 30. A surface 30a opposite to the surface 30b is the upper surface 30a of the package 30.

For example, the package 30 includes a multilayer board. The package 30 includes, for example, multiple dielectric layers and multiple wiring layers. The wiring layers are located on the dielectric layers, and the dielectric layers are located between the wiring layers. The wiring layers include multiple wires. The wires include wires in the multiple layers. The dielectric layers in the package 30 may be formed from a ceramic material or an organic material. Examples of the ceramic material include sintered aluminum oxide and sintered mullite. Examples of the organic material include an epoxy resin and a polyimide resin. The wires in the package 30 are formed from, for example, a metal. The wires in the package 30 may be formed from copper, gold, or another metal. The wires in the package 30 are metal wires. The package 30 includes multiple vias 300 that connect the wires in the multiple layers to each other. The package 30 includes multiple connection pads 310.

The package 30 includes a bottom 340, a peripheral wall (also referred to as a side wall) 341 on the bottom 340, and a partition 342 that divides a space surrounded by the peripheral wall 341 into two spaces. The two spaces resulting from the partition 342 dividing the space surrounded by the peripheral wall 341 are recesses 331 and 332. The lower surface of the bottom 340 is the lower surface 30b of the package 30.

The recess 331 accommodates the light emitter 31. The recess 332 accommodates the light receiver 32. The recesses 331 and 332 are each open toward the upper surface 30a. The recesses 331 and 332 are not connected to each other and are apart from each other. The light emitter 31 is located on the bottom of the recess 331. The light receiver 32 is located on the bottom of the recess 332.

The recess 331 accommodating the light emitter 31 has a step on its inner peripheral surface. One of the connection pads 310 (also referred to as a first connection pad 310) is located on an upper surface 331a of the step (refer to FIG. 6). A bonding wire 33 electrically connects one of a positive terminal or a negative terminal in the light emitter 31 to the first connection pad 310. Two of the connection pads 310 are located on the lower surface 30b of the package 30. One of the two connection pads 310 is electrically connected to the positive terminal in the light emitter 31, and the other of the two connection pads 310 is electrically connected to the negative terminal in the light emitter 31. The vias 300 and the wires electrically connect one of the two connection pads 310 to the first connection pad 310. The vias 300 and the wires electrically connect the other of the two connection pads 310 to the other of the positive terminal or the negative terminal in the light emitter 31. A conductive bond such as solder connects the connection pad 310 that is electrically connected to the negative terminal in the light emitter 31 and is located on the lower surface 30b of the package 30 to the connection pads 22 that are located on the surface 2a of the wiring board 2 and are electrically connected to the ground wire 20. Thus, the negative terminal in the light emitter 31 is electrically connected to the ground wire 20. A conductive bond such as solder connects the connection pad 310 that is electrically connected to the positive terminal in the light emitter 31 and is located on the lower surface 30b of the package 30 to the connection pads 22 that are located on the surface 2a of the wiring board 2 and are electrically connected to the processing circuit 7. Thus, the positive terminal in the light emitter 31 is electrically connected to the processing circuit 7 that controls emission of the light emitter 31.

The recess 332 accommodating the light receiver 32 has a step on its inner peripheral surface. One of the connection pads 310 (also referred to as a second connection pad 310) is located on an upper surface 332a of the step (refer to FIG. 6). The bonding wire 33 electrically connects one of an output terminal or a negative terminal in the light receiver 32 to the second connection pad 310. Two of the connection pads 310 are located on the lower surface 30b of the package 30. One of the two connection pads 310 is electrically connected to the output terminal in the light receiver 32, and the other is electrically connected to the negative terminal in the light receiver 32. The vias 300 and the wires electrically connect one of the two connection pads 310 to the second connection pad 310. The vias 300 and the wires electrically connect the other of the two connection pads 310 to the other of the output terminal or the negative terminal in the light receiver 32. A conductive bond such as solder connects the connection pad 310 that is electrically connected to the negative terminal in the light receiver 32 and is located on the lower surface 30b of the package 30 to the connection pads 22 that are located on the surface 2a of the wiring board 2 and are electrically connected to the ground wire 20. Thus, the negative terminal in the light receiver 32 is electrically connected to the ground wire 20. A conductive bond such as solder connects the connection pad 310 that is electrically connected to the output terminal in the light receiver 32 and is located on the lower surface 30b of the package 30 to the connection pads 22 that are located on the surface 2a of the wiring board 2 and are electrically connected to the amplifier 4. Thus, the output terminal in the light receiver 32 is electrically connected to the amplifier 4. The amplifier 4 amplifies and outputs the output signal from the output terminal in the light receiver 32.

The package 30 includes a wire 320 (also referred to as a first wire 320) that is electrically connected to the ground wire 20 in the wiring board 2. The first wire 320 surrounds the light emitter 31 and the light receiver 32 in a plan view or in a transparent plan view of the package 30.

The wire 320 includes, for example, a wire including multiple layers (also referred to as a second multilayer wire). In the present embodiment, the second multilayer wire includes an outer-layer wire 321 on the upper surface 30a of the package 30 and an inner-layer wire 322 including multiple layers inside the package 30. The outer-layer wire 321 and the inner-layer wire 322 including multiple layers are electrically connected to the ground wire 20.

The outer-layer wire 321 surrounds the light emitter 31 and the light receiver 32 in a plan view from above (in other words, the upper surface 30a of) the package 30 as shown in FIG. 6. The outer-layer wire 321 is located on upper surfaces of the peripheral wall 341 and the partition 342 in the package 30. The outer-layer wire 321 surrounds the light emitter 31 and the light receiver 32 in a plan view. In other words, the outer-layer wire 321 separately surrounds each of the light emitter 31 and the light receiver 32 in a plan view. The outer-layer wire 321 separately surrounds each of the recesses 331 and 332 in a plan view. The outer-layer wire 321 is located above the light emitter 31 and the light receiver 32. The outer-layer wire 321 may not be located on the upper surface of the partition 342 and may collectively surround the light emitter 31 and the light receiver 32.

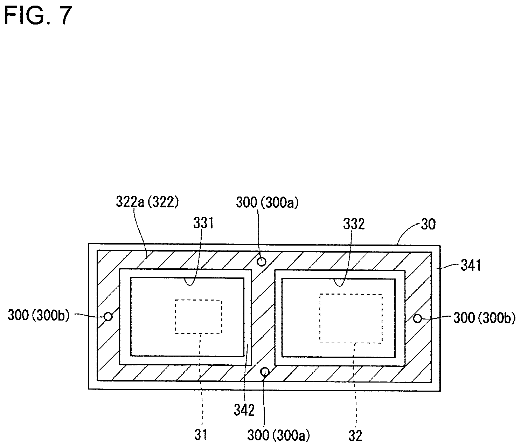

FIG. 7 is a schematic plan view of an example inner-layer wire 322a included in the inner-layer wire 322 including multiple layers. FIG. 7 shows the inner-layer wire 322a and a part below the inner-layer wire 322a in the package 30. In FIG. 7, dashed lines represent the light emitter 31 and the light receiver 32 when the package 30 is viewed from above.

As shown in FIG. 7, the inner-layer wire 322a is located inside the peripheral wall 341 and the partition 342 in the package 30. The inner-layer wire 322a surrounds the recesses 331 and 332 in a plane parallel to the lower surface 30b of the package 30. The inner-layer wire 322a surrounds the light emitter 31 and the light receiver 32 in a transparent plan view of the package 30. In the present embodiment, the inner-layer wire 322a separately surrounds each of the light emitter 31 and the light receiver 32 in a transparent plan view of the package 30.

The inner-layer wire 322a may be located above at least one of the light emitter 31 or the light receiver 32. The inner-layer wire 322a may be located below at least one of the light emitter 31 or the light receiver 32. The inner-layer wire 322a may be located between the upper surface and the lower surface of the light emitter 31 or between the upper surface and the lower surface of the light receiver 32 in the vertical direction of the measurement device 1. The inner-layer wire 322a may surround the light emitter 31 and the light receiver 32 in a plane parallel to the lower surface 30b of the package 30. The inner-layer wire 322a may not be located inside the partition 342 and may collectively surround the light emitter 31 and the light receiver 32.

Each of the layers of the inner-layer wire 322 in the package 30 may have the same structure as the structure shown in FIG. 7. A part of the layers of the inner-layer wire 322 alone may have the same structure as the structure shown in FIG. 7.

The package 30 includes a wire 325 (also referred to as a third wire 325) electrically connected to the ground wire 20 in the wiring board 2, in addition to the wire 320 including the outer-layer wire 321 and the inner-layer wire 322. The third wire 325 is, for example, an inner-layer wire inside the package 30. The third wire 325 is included in, for example, an undermost wiring layer of the multiple wiring layers in the package 30. The third wire 325 is located below the inner-layer wire 322. The third wire 325 is located inside the bottom 340 of the package 30.

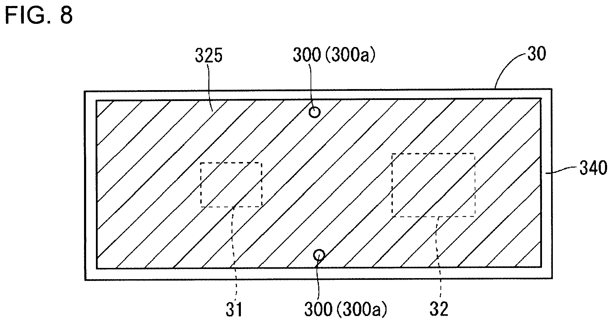

FIG. 8 is a schematic plan view of the wire 325. FIG. 8 shows the wire 325 and a part below the wire 325 in the package 30. In FIG. 8, dashed lines represent the light emitter 31 and the light receiver 32 when the package 30 is viewed from above. The wire 325 extends in a planar manner along the lower surface 30b of the package 30 as shown in FIGs. 3 and 8. The wire 325 is located closer to the wiring board 2 than to the light emitter 31 and to the light receiver 32. The light emitter 31 and the light receiver 32 are located above the wire 325. In detail, the light emitter 31 and the light receiver 32 are located immediately above the wire 325. The wire 325 is also referred to as a solid wire. The wire 325 may include a planar wire immediately above which the light emitter 31 is located and a planar wire immediately above which the light receiver 32 is located. In this case, the planar wires are not connected to each other, and the planar wires may be separately connected to different ground wires or to a single ground wire.

The vias 300 in the package 30 include multiple through-hole vias 300a that extend from the upper surface 30a to the lower surface 30b and are electrically connected to the ground wire 20. The vias 300 in the package 30 include multiple buried vias 300b that are electrically connected to the ground wire 20. Each of the buried vias 300b is connected to the inner-layer wire 322. At least one of the buried vias 300b may be connected to at least two layers of the inner-layer wire 322 of the multiple layers of the inner-layer wire 322. Each of the through-hole vias 300a is connected to the outer-layer wire 321, the inner-layer wire 322, and the wire 325. Each of the through-hole vias 300a is connected to the connection pad 310 on the lower surface 30b of the package 30. A conductive bond such as solder connects this connection pad 310 to connection pads 22 that are located on the surface 2a of the wiring board 2 and are electrically connected to the ground wire 20. The outer-layer wire 321, the inner-layer wire 322, and the wire 325 are electrically connected to the ground wire 20.

The structure of the wire 320 is not limited to the above structure. For example, the wire 320 may not surround the light emitter 31 and the light receiver 32 in a plan view from above the package 30. In this case, the wire 320 may include no outer-layer wire 321. The wire 320 may not surround the light emitter 31 and the light receiver 32 in a transparent plan view of the package 30. In this case, the wire 320 may include no inner-layer wire 322a.

The wire 320 may include a wire including multiple layers that surround the light emitter 31 and the light receiver 32 in a transparent plan view of the package 30 in a cooperative manner. In this case, the wire 320 may or may not include a wire that surrounds the light emitter 31 and the light receiver 32 alone, like the outer-layer wire 321 and the inner-layer wire 322a as shown in FIG. 6.

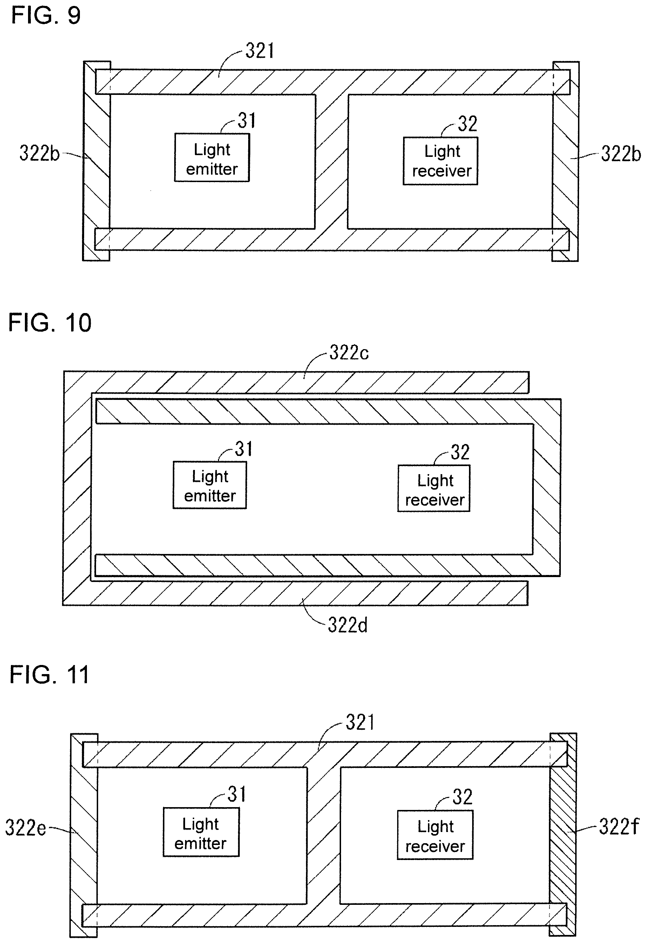

FIG. 9 is a schematic plan view of an example wire including multiple layers that surround the light emitter 31 and the light receiver 32 in a transparent plan view of the package 30 in a cooperative manner. FIG. 9 shows the outer-layer wire 321 and an inner-layer wire 322b included in the inner-layer wire 322 as viewed from above the upper surface 30a. FIG. 9 shows the outer-layer wire 321 and the inner-layer wire 322b alone, among the components in the package 30. In FIG. 9, the outer-layer wire 321 and the inner-layer wire 322b surround the light emitter 31 and the light receiver 32 in a cooperative manner, although each of the outer-layer wire 321 and the inner-layer wire 322b does not surround the light emitter 31 and the light receiver 32 alone.

The inner-layer wire 322b may be located above at least one of the light emitter 31 or the light receiver 32. The inner-layer wire 322b may be located below at least one of the light emitter 31 or the light receiver 32. The inner-layer wire 322b may be located between the upper surface and the lower surface of the light emitter 31 or between the upper surface and the lower surface of the light receiver 32 in the vertical direction of the measurement device 1.

FIG. 10 is a schematic plan view of an example wire including multiple layers that surround the light emitter 31 and the light receiver 32 in a transparent plan view of the package 30 in a cooperative manner. FIG. 10 shows inner-layer wires 322c and 322d included in the inner-layer wire 322 as viewed from above the upper surface 30a. FIG. 10 shows the inner-layer wires 322c and 322d alone, among the components in the package 30. The inner-layer wire 322c is included in a wiring layer different from the inner-layer wire 322d. In FIG. 10, the inner-layer wires 322c and 322d surround the light emitter 31 and the light receiver 32 in a cooperative manner, although each of the inner-layer wires 322c and 322d does not surround the light emitter 31 and the light receiver 32 alone.

At least one of the inner-layer wire 322c or 322d may be located above at least one of the light emitter 31 or the light receiver 32. At least one of the inner-layer wire 322c or 322d may be located below at least one of the light emitter 31 or the light receiver 32. At least one of the inner-layer wire 322c or 322d may be located between the upper surface and the lower surface of the light emitter 31 or between the upper surface and the lower surface of the light receiver 32 in the vertical direction of the measurement device 1.

FIG. 11 is a schematic plan view of an example wire including multiple layers that surround the light emitter 31 and the light receiver 32 in a transparent plan view of the package 30 in a cooperative manner. FIG. 11 shows the outer-layer wire 321 and inner-layer wires 322e and 322f included in the multiple layers of the inner-layer wire 322 as viewed from above the upper surface 30a. FIG. 11 shows the outer-layer wire 321 and the inner-layer wires 322e and 322f alone, among the components in the package 30. The inner-layer wire 322e is included in a wiring layer different from the inner-layer wire 322f. In FIG. 11, the outer-layer wire 321 and the inner-layer wires 322e and 322f surround the light emitter 31 and the light receiver 32 in a cooperative manner, although each of the outer-layer wire 321 and the inner-layer wires 322e and 322f does not surround the light emitter 31 and the light receiver 32 alone.

At least one of the inner-layer wire 322e or 322f may be located above at least one of the light emitter 31 or the light receiver 32. At least one of the inner-layer wire 322e or 322f may be located below at least one of the light emitter 31 or the light receiver 32. At least one of the inner-layer wire 322e or 322f may be located between the upper surface and the lower surface of the light emitter 31 or between the upper surface and the lower surface of the light receiver 32 in the vertical direction of the measurement device 1.

The wire including multiple layers in the package 30 that surrounds the light emitter 31 and the light receiver 32 in a transparent plan view of the package 30 is not limited to the above manner. For example, a wire including four or more layers in the wire 320 may surround the light emitter 31 and the light receiver 32 in a cooperative manner.

Example Structure of Cover

As shown in FIGs. 2 and 3, the cover 38 is fixed on the upper surface 30a of the package 30 to cover the recesses 331 and 332 in the package 30. The cover 38 includes, for example, a light-transmissive base 380 and a light-shielding film 381 formed from a metal. The cover 38 is, for example, rectangular in a plan view. The light-shielding film 381 is located on the lower surface of the base 380. The light-shielding film 381 has openings 381a and 381b extending in the thickness direction. The opening 381a faces a part of the opening of the recess 331. The opening 381b faces a part of the opening of the recess 332. Thus, the light-shielding film 381 covers a part of the recess 331 and a part of the recess 332. The illumination target 500 is illuminated with the light L1 that is emitted from the light emitter 31 in the recess 331 and passes through the opening 381a in the light-shielding film 381. The light receiver 32 in the recess 332 receives the coherent light L2 that includes light scattered by the illumination target 500 and passes through the opening 381b in the light-shielding film 381. The light-shielding film 381 may block 97% or more of light, for example. The light-shielding film 381 may block 99% or more of light.

The light-transmissive base 380 is formed from, for example, a transparent material. Examples of the material for the base 380 include glass, a ceramic, and a resin. The light-shielding film 381 may be formed from, for example, a metal such as Cr, Ti, Al, Cu, Co, Ag, Au, Pd, Pt, Ru, Sn, Ta, Fe, In, Ni, or W. In some embodiments, the light-shielding film 381 may be formed from an alloy of two or more metals of Cr, Ti, Al, Cu, Co, Ag, Au, Pd, Pt, Ru, Sn, Ta, Fe, In, Ni, and W.

For example, a conductive bond such as solder connects the light-shielding film 381 to the outer-layer wire 321 on the upper surface 30a of the package 30. Thus, the light-shielding film 381 is electrically connected to the ground wire 20 in the wiring board 2.

The measurement device 1 includes the light-shielding film 381 that covers a part of the recess 331 accommodating the light emitter 31 and a part of the recess 332 accommodating the light receiver 32. The light-shielding film 381 can direct the light L1 emitted from the light emitter 31 in the recess 331 in an intended direction. The light receiver 32 in the recess 332 can receive light in an intended direction. Thus, the calculator 6 can determine the flow calculation value as appropriate based on the output signal from the light receiver 32. This improves the precision of the flow calculation value (at least one of the flow velocity or the flow rate) determined by the calculator 6.

In FIGs. 2 and 3, the openings 381a and 381b in the light-shielding film 381 are filled with the base 380. However, the openings 381a and 381b may not be filled with the base 380. The measurement device 1 may include no cover 38.

Relationship between Outline of Shield Case and Outline of Package

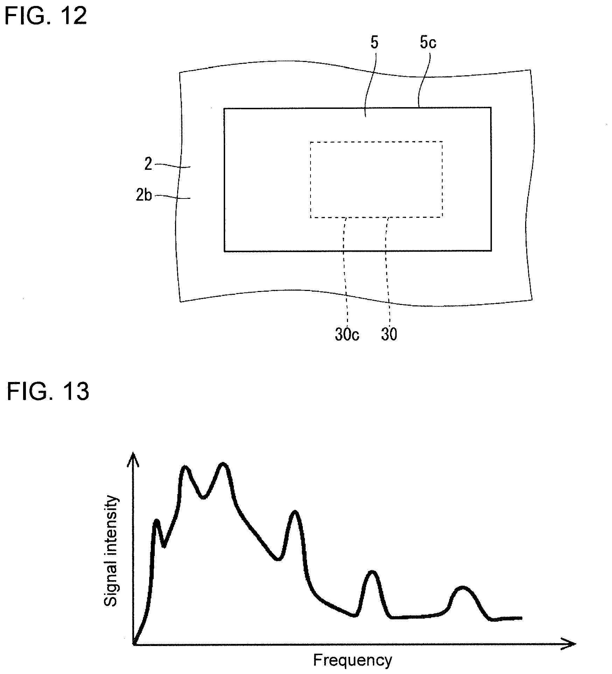

FIG. 12 is a plan view of the shield case 5 covering the amplifier 4 and the package 30 in the sensor 3, showing an example relationship of the outline of the shield case 5 and the outline of the package 30. FIG. 12 schematically shows the shield case 5 and the wiring board 2 in a plan view from below. In the present embodiment, an outline 5c of the shield case 5 is located outward from an outline 30c of the package 30 in a transparent plan view, as shown in FIG. 12. In other words, the entire region of the package 30 overlaps the shield case 5 in a transparent plan view. Thus, the area of the shield case 5 mounted on the surface 2b of the wiring board 2 is larger than the area of the package 30 mounted on the surface 2a of the wiring board 2.

The outline 5c of the shield case 5 may not be located outward from the outline 30c of the package 30 in a transparent plan view. In this case, at least a part of the package 30 may overlap the shield case 5 in a transparent plan view.

In the measurement device 1 with the structure described above, when ambient noise affects at least one of the light emitter 31, the light receiver 32, or the amplifier 4, the calculator 6 may inappropriately determine the flow calculation value. This may lower the precision of the flow calculation value (at least one of the flow velocity or the flow rate) determined by the calculator 6. A source of ambient noise around the light emitter 31, the light receiver 32, and the amplifier 4 may be, for example, the calculator 6. When a motor drives a pump or another device around the measurement device 1, the motor may be a source of noise.

FIG. 13 is a graph showing an example power spectrum that is determined by the calculator 6 when ambient noise affects at least one of the light emitter 31, the light receiver 32, or the amplifier 4. When ambient noise affects at least one of the light emitter 31, the light receiver 32, or the amplifier 4, the waveform of the power spectrum may be disturbed as shown in FIG. 13. In this case, the calculator 6 may inappropriately determine the flow calculation value.

In the present embodiment, the wire 320 surrounding the light emitter 31 and the light receiver 32 in a plan view or in a transparent plan view of the package 30 and the shield case 5 covering the amplifier 4 are electrically connected to the ground wire 20 in the wiring board 2. Thus, the light emitter 31, the light receiver 32, and the amplifier 4 are less susceptible to ambient noise. More specifically, the noise immunity of the measurement device 1 can be improved. Thus, the calculator 6 can appropriately determine the flow calculation value.

When the wire 320 surrounding the light emitter 31 and the light receiver 32 in a plan view or in a transparent plan view of the package 30 includes the wire including multiple layers that is electrically connected to the ground wire 20, the light emitter 31 and the light receiver 32 are still less susceptible to ambient noise. Thus, the calculator 6 can more appropriately determine the flow calculation value.

When the light emitter 31 and the light receiver 32 are located immediately above the planar wire 325 electrically connected to the ground wire 20, the light emitter 31 and the light receiver 32 are still less susceptible to ambient noise. Thus, the calculator 6 can more appropriately determine the flow calculation value.

When the measurement device 1 includes the light-shielding film 381 of a metal that is electrically connected to the ground wire 20 and covers a part of the recess 331 and a part of the recess 332, the light emitter 31 in the recess 331 and the light receiver 32 in the recess 332 are still less susceptible to ambient noise. Thus, the calculator 6 can more appropriately determine the flow calculation value.

In the example of FIG. 12 described above, the outline 5c of the shield case 5 on the surface 2b of the wiring board 2 is located outward from the outline 30c of the package 30 in the sensor 3 on the surface 2a of the wiring board 2. In other words, the entire region of the package 30 overlaps the shield case 5 in a transparent plan view. In this case, noise generated from below (in other words, the surface 2b of) the wiring board 2 is less likely to reach the light emitter 31 and the light receiver 32 on the wiring board 2. For example, noise generated from the calculator 6 on the surface 2b is less likely to reach the light emitter 31 and the light receiver 32 on the surface 2a. When a noise source is below the measurement device 1, noise from the noise source is less likely to reach the light emitter 31 and the light receiver 32 on the surface 2a. The light emitter 31 and the light receiver 32 are still less susceptible to ambient noise.

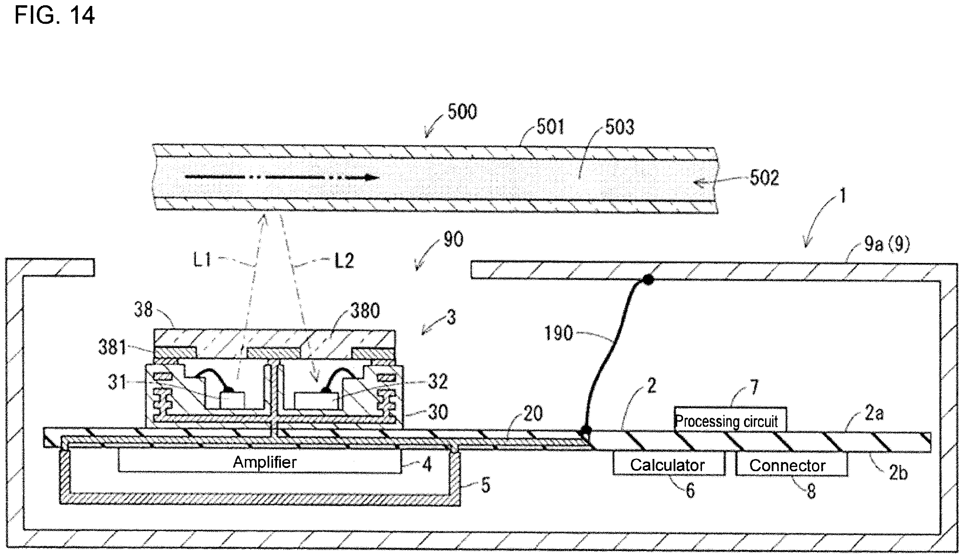

In the above embodiment, the outer case 9 is formed from a resin. However, the outer case 9 may be formed from, for example, a metal to function as a shield case 9a. In this case, the shield case 9a may be electrically connected to the ground wire 20 in the wiring board 2. FIG. 14 is a view of an example electrical connection of the shield case 9a to the ground wire 20. The shield case 9a may not be electrically connected to the ground wire 20.

In FIG. 14, an electric wire 190 electrically connected to the ground wire 20 is connected to the shield case 9a. A conductive bond such as solder connects one end of the electric wire 190 to, for example, a corresponding connection pad 22 on the surface 2a of the wiring board 2 and electrically connected to the ground wire 20. A conductive bond such as solder connects the other end of the electric wire 190 to, for example, the shield case 9a. The shield case 9a is formed from, for example, a metal such as aluminum or stainless steel.

Thus, when the measurement device 1 includes the shield case 9a covering, for example, the wiring board 2, the sensor 3, the amplifier 4, the shield case 5, and the calculator 6, the light emitter 31, the light receiver 32, and the amplifier 4 are still less susceptible to ambient noise. In other words, the noise immunity of the measurement device 1 is improved further. Thus, the calculator 6 can appropriately determine the flow calculation value further.

When the shield case 9a is electrically connected to the ground wire 20 in the wiring board 2 as shown in FIG. 14, the light emitter 31, the light receiver 32, and the amplifier 4 are still less susceptible to ambient noise. Thus, the calculator 6 can appropriately determine the flow calculation value further.

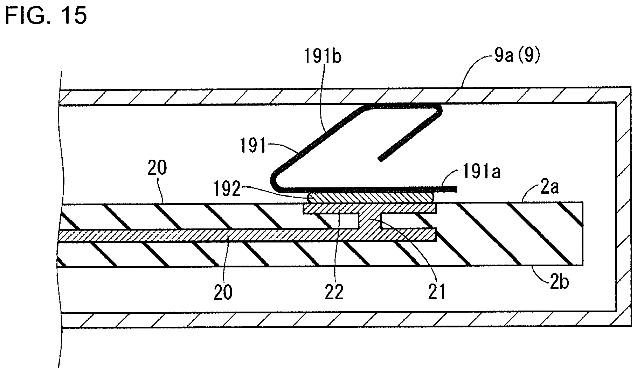

The shield case 9a may include a base formed from a resin with a surface covered with a metal film. This metal film is formed from, for example, nickel. A method for electrically connecting the shield case 9a to the ground wire 20 is not limited to the above example. For example, a leaf spring 191 may electrically connect the shield case 9a to the ground wire 20. FIG. 15 is a view of an example electrical connection of the shield case 9a to the ground wire 20 with the leaf spring 191.

The leaf spring 191 is formed from, for example, a metal. The leaf spring 191 includes, for example, a fastener 191a fixed on the wiring board 2 and a contact part 191b in contact with the shield case 9a. A conductive bond 192 such as solder connects the fastener 191a to, for example, a corresponding connection pad 22 on the surface 2a of the wiring board 2. The vias 21 electrically connect the connection pads 22 to the ground wire 20. The contact part 191b is in contact with the inner surface of the shield case 9a. In the example of FIG. 15 as well, the light emitter 31, the light receiver 32, and the amplifier 4 are less susceptible to ambient noise.

The measurement device 1 has been described in detail as above, but the above structures are illustrative in all respects, and the disclosure is not limited to the above structures. The above embodiments may be combined in any manner unless any contradiction arises. Examples other than those illustrated above may also be included without departing from the scope of the present disclosure.

Reference Signs List

1 measurement device
2 wiring board
2a, 2b first surface, second surface
3 sensor
4 amplifier
5, 9a shield case
6 calculator
20 ground wire
30 package
31 light emitter
32 light receiver
320, 325 wire (first wire, third wire)
321 outer-layer wire (second wire)
322 inner-layer wire (second wire)
331,332 recess
381 light-shielding film

**Claims**

1. A measurement device, comprising:

   a wiring board having a first surface and a second surface opposite to the first surface, the wiring board including a ground wire;
   a sensor on the first surface;
   a calculator;
   an amplifier on the second surface; and
   a first shield case covering the amplifier,
   wherein the sensor includes

      a light emitter configured to illuminate, with light, an illumination target having an internal space in which a fluid flows,
      a light receiver configured to receive coherent light including light scattered by the illumination target in the light from the light emitter, and to output an output signal corresponding to intensity of the coherent light, and
      a package accommodating the light emitter and the light receiver and including a first wire,

   the amplifier amplifies the output signal,
   the calculator calculates a calculation value indicating a flow state of the fluid based on the output signal amplified by the amplifier,
   the first wire surrounds the light emitter and the light receiver in a plan view or in a transparent plan view of the package, and
   the first wire and the first shield case are electrically connected to the ground wire.

2. The measurement device according to claim 1, wherein
   the first wire includes a second wire including a plurality of layers.

3. The measurement device according to claim 2, wherein
   the package includes a via connected to at least two layers of the plurality of layers of the second wire.

4. The measurement device according to any one of claims 1 to 3, wherein

   the package includes a third wire electrically connected to the ground wire and extending in a planar manner, and
   the light emitter and the light receiver are located on the third wire in a transparent plan view of the package.

5. The measurement device according to any one of claims 1 to 4, further comprising:
   a second shield case covering the wiring board, the sensor, the calculator, the amplifier, and the first shield case.

6. The measurement device according to claim 5, wherein
   the second shield case is electrically connected to the ground wire.

7. The measurement device according to any one of claims 1 to 6, wherein
   the first shield case has an outline located outward from an outline of the package in the sensor in a transparent plan view.

8. The measurement device according to any one of claims 1 to 7, wherein

   the package includes

      a first recess receiving the light emitter, and
      a second recess receiving the light receiver,

   the measurement device further comprises a light-shielding film comprising a metal and covering a part of the first recess and a part of the second recess, and
   the light-shielding film is electrically connected to the ground wire.

9. The measurement device according to any one of claims 1 to 8, wherein

the ground wire is located in an inner layer of the wiring board.

FIG. 1

FIG. 2

FIG. 3

EP 4 098 980 A1

FIG. 4

FIG. 5

FIG. 6

FIG. 7

FIG. 8

## FIG. 9

## FIG. 10

## FIG. 11

## FIG. 12

## FIG. 13

FIG. 14

EP 4 098 980 A1

FIG. 15

<div style="text-align:center">

**INTERNATIONAL SEARCH REPORT**

</div>

| International application No. |
| --- |
| PCT/JP2021/002743 |

**A. CLASSIFICATION OF SUBJECT MATTER**
Int.Cl. G01F1/66(2006.01)i, A61B5/026(2006.01)i, A61B5/0285(2006.01)i,
G01F1/00(2006.01)i
FI: G01F1/66103, G01F1/00Q, A61B5/026120, A61B5/0285H
According to International Patent Classification (IPC) or to both national classification and IPC

**B. FIELDS SEARCHED**

Minimum documentation searched (classification system followed by classification symbols)
Int.Cl. G01F1/66, A61B5/02-5/03, G01F1/00, G01P5/26

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2019-141470 A (FUJI XEROX CO., LTD.) 29 August 2019 (2019-08-29), paragraphs [0042]-[0052], [0074]-[0106], fig. 3-5, 9-13 | 1-9 |
| Y | JP 2002-122474 A (HOCHIKI CORP.) 26 April 2002 (2002-04-26), paragraphs [0012], [0022]-[0055], fig. 1-7 | 1-9 |
| Y | WO 2017/175504 A1 (KYOCERA CORPORATION) 12 October 2017 (2017-10-12), paragraphs [0114]-[0165], fig. 16-26 | 2-9 |
| Y | JP 2018-196571 A (KYOCERA CORPORATION) 13 December 2018 (2018-12-13), paragraphs [0041]-[0047], fig. 1, 3 | 8-9 |

☐ Further documents are listed in the continuation of Box C.  ☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 02 March 2021 | 16 March 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

Information on patent family members

| International application No. |
| --- |
| PCT/JP2021/002743 |

```
JP 2019-141470 A   29 August 2019     (Family: none)

JP 2002-122474 A   26 April 2002      (Family: none)

WO 2017/175504 A1  12 October 2017       JP 6616496 B2
                                         US 2019/0110697 A1
                                         paragraphs [0140]-[0191], fig. 16-26
                                         JP 2020-58813 A
                                         EP 3440998 A1
                                         CN 109069043 A

JP 2018-196571 A   13 December 2018      (Family: none)
```

Form PCT/ISA/210 (patent family annex) (January 2015)

**EP 4 098 980 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6350280 A

- JP 10233471 A